# EUROPEAN PATENT SPECIFICATION

(11) **EP 3 368 156 B1**
(45) Date of publication and mention of the grant of the patent: **12.02.2020**
(21) Application number: 16809580.0
(22) Date of filing: 15.11.2016
(51) Int. Cl.: A61N 7/00, A61B 18/00

(54) **BLOOD VESSEL ISOLATION ABLATION DEVICE**
BLUTGEFÄSSISOLATIONABLATIONSVORRICHTUNG
DISPOSITIF D'ISOLEMENT ET D'ABLATION DE VAISSEAU SANGUIN

(30) Priority: 10.12.2015 US 201562265739 P
(43) Date of publication of application: 05.09.2018
(73) Proprietor: St. Jude Medical, Cardiology Division, Inc., St. Paul, MN 55117 (US)
(72) Inventor: LUPOTTI, Fermin A., Lake Forest, CA 92630 (US); TEGG, Troy T., Elk River, MN 55330 (US)
(74) Representative: Kramer Barske Schmidtchen Patentanwälte PartG mbB
(86) International application number: PCT/US2016/061980
(87) International publication number: WO 2017/099950

(56) References cited:
- WO-A1-99/02096
- US-A1- 2002 019 627
- US-A1- 2002 173 784
- US-A1- 2003 120 270
- US-A1- 2005 065 504
- US-A1- 2014 257 266

## Description

### BACKGROUND

The instant disclosure relates generally to tissue ablation. In particular, the instant disclosure relates to a medical device for use in creating a circumferential lesion about a blood vessel, such as in pulmonary vein (PV) isolation procedures.

In the normal heart, contraction and relaxation of the heart muscle (myocardium) takes place in an organized fashion as electro-chemical signals pass sequentially through the myocardium from the sinoatrial (SA) node, which comprises a bundle of unique cells disposed in the wall of the right atrium, to the atrioventricular (AV) node and then a long a well-defined route, which includes the His-Purkinje system, into the left and right ventricles. Sometimes, however, abnormal rhythms occur in the heart, which are referred to generally as arrhythmias.

Certain arrhythmias, referred to as atrial arrhythmias, occur in the atria. Three of the most common atrial arrhythmias are ectopic atrial tachycardia, atrial fibrillation (AF), and atrial flutter. AF can result in significant patient discomfort and even death because of a number of associated problems, including: irregular heart rate, which causes patient discomfort and anxiety; loss of synchronous atrioventricular contractions, which compromises cardiac hemodynamics, resulting in varying levels of congestive heart failure; and stasis of blood flow, which increases the likelihood of thromboembolism, a leading cause of stroke.

One common medical procedure for the treatment of certain types of cardiac arrhythmia, specifically including AF, is catheter ablation. In many ablation procedures, energy, such as radiofrequency (RF) or high intensity focused ultrasound (HIFU) energy, is delivered to cardiac tissue in order to heat the tissue and create a permanent scar or lesion that is electrically inactive.

It is known that, in some instances, stray electrical signals find a pathway down the pulmonary veins and into the left atrium of the heart. In these instances, it may be advantageous to produce a circumferential lesion at or near the ostium of one or more of the pulmonary veins. Desirably, such a circumferential lesion would electrically isolate the pulmonary vein from the left atrium, completely blocking stray signals from traveling down the pulmonary vein and into the left atrium. Thus, such procedures are known as PV isolation procedures.

WO 99/02096 A1 discloses a circumferential ablation device assembly and method.

US 2014/0257266 A1 discloses a balloon catheter apparatus with a microwave emitter.

### BRIEF SUMMARY

The invention provides an ablation system according to claim 1. Further aspects and preferred embodiments are defined in the dependent claims. Aspects, embodiments, examples, and methods of the present disclosure that do not fall within the scope of the appended claims do not form part of the invention and are merely provided for illustrative purposes.

Disclosed herein is an ablation device, including: an elongate catheter body having a lumen extending therethrough, the elongate catheter body including a distal region; an ablation element disposed within the distal region, wherein the ablation element is configured to emit ablative energy outwardly relative to a longitudinal axis of the elongate catheter body; and a balloon positioned about the distal region and defining an interior in communication with the lumen, wherein the balloon is expandable outwardly from an outer surface of the elongate catheter body, and wherein, when the balloon is expanded outwardly from the outer surface of the elongate catheter body, an exterior surface of the balloon is shaped to stably engage an interior wall of a blood vessel without occluding blood flow through the blood vessel. The balloon can have a cross-shaped transverse cross-section when expanded.

In embodiments, the ablation element is an acoustic ablation element, such as a plurality of ultrasound transducers arranged in a circumferential array, a plurality of ultrasound transducers arranged in a linear array, and/or a plurality of ultrasound transducers arranged in both a linear array and a circumferential array (*e.g.*, a linear array of circumferential arrays of ultrasound transducers). The ablation element can be omnidirectional or unidirectional.

According to aspects of the disclosure, the ablation element is disposed within the balloon. The ablation element can also be mounted within the distal region such that it can move along a longitudinal axis of the elongate catheter body and/or so that it can rotate about a longitudinal axis of the elongate catheter body.

It is also contemplated that the ablation element can be operated in an imaging or monitoring mode, for example to image and/or monitor the progress of an ablation lesion forming in the tissue.

According to other aspects disclosed herein, an ablation system includes: an ablation catheter including: an elongate catheter body having a lumen extending therethrough, the elongate catheter body including a distal region; an ultrasound transducer disposed within the distal region, wherein the ultrasound transducer is configured to emit acoustic energy outwardly relative to a longitudinal axis of the elongate catheter body and to receive acoustic energy inwardly relative to the longitudinal axis of the elongate catheter body; and a balloon positioned about the distal region and defining an interior in communication with the lumen, wherein the balloon is expandable outwardly from an outer surface of the elongate catheter body, and wherein, when the balloon is expanded outwardly from the outer surface of the elongate catheter body, an exterior surface of the balloon is shaped to stably engage an interior wall of a blood vessel without occluding blood flow through the blood vessel; and a control unit configured to operate the ultrasound transducer in an ablating mode, wherein the ultrasound transducer emits acoustic energy at an ablation level, and in an imaging mode, wherein the ultrasound transducer emits acoustic energy at an imaging level and receives acoustic energy echoes from adjacent tissue. The control unit can also be configured to electronically steer a focus of the acoustic energy emitted by the ultrasound transducer.

Several embodiments of the ultrasound transducer are contemplated. For example, the ultrasound transducer can include a circumferential array of acoustic elements. In other embodiments, the ultrasound transducer can be mounted within the distal region such that it can rotate about a longitudinal axis of the elongate catheter body, for example to ablate, image, and/or monitor tissue 360 degrees about the circumference of the catheter body. In still other embodiments, the ultrasound transducer can include an omnidirectional ultrasound transducer.

It is also contemplated that the control unit is further configured to operate the ultrasound transducer in the ablating mode to create a circumferential lesion in a wall of a blood vessel surrounding the distal region.

According to additional aspects of the disclosure, the ultrasound transducer includes both a linear array of omnidirectional acoustic elements and a unidirectional acoustic element mounted within the distal region such that the unidirectional acoustic element can rotate about a longitudinal axis of the elongate catheter body. The control unit can be configured to operate the linear array of omnidirectional acoustic elements in the ablating mode and to operate the unidirectional acoustic element in the imaging mode.

Also disclosed herein is a method of ablating a blood vessel. The method includes: introducing an ablation catheter into a patient's vasculature, the ablation catheter including: an elongate catheter body having a lumen extending therethrough, the elongate catheter body including a distal region; an ultrasound transducer disposed within the distal region, wherein the ultrasound transducer is configured to emit acoustic energy outwardly relative to a longitudinal axis of the elongate catheter body; and a balloon positioned about the distal region and defining an interior in communication with the lumen, wherein the balloon is expandable outwardly from an outer surface of the elongate catheter body; positioning the ablation catheter within the blood vessel; causing the balloon to expand outwardly from the outer surface of the elongate catheter body such that an exterior surface of the balloon stably engages a wall of the blood vessel without occluding blood flow through the blood vessel; and causing the ultrasound transducer to emit acoustic energy at an ablating level such that the ultrasound transducer creates a first circumferential lesion about the blood vessel without repositioning the ablation catheter.

The ultrasound transducer can include a circumferential array of acoustic elements. Causing the ultrasound transducer to emit acoustic energy at an ablating level such that the ultrasound transducer creates a first circumferential lesion about the blood vessel without repositioning the ablation catheter can include activating each acoustic element of the circumferential array of acoustic elements to ablate about a circumference of the blood vessel.

In other embodiments, the ultrasound transducer can include an acoustic element mounted such that the acoustic element can rotate about a longitudinal axis of the elongate catheter body. Similarly, causing the ultrasound transducer to emit acoustic energy at an ablating level such that the ultrasound transducer creates a first circumferential lesion about the blood vessel without repositioning the ablation catheter can include activating the acoustic element while it rotates about the longitudinal axis of the elongate catheter body to ablate about a circumference of the blood vessel.

In still other embodiments, the ultrasound transducer can include an omnidirectional acoustic element. Similarly, causing the ultrasound transducer to emit acoustic energy at an ablating level such that the ultrasound transducer creates a first circumferential lesion about the blood vessel without repositioning the ablation catheter can include activating the omnidirectional acoustic element to ablate about a circumference of the blood vessel.

According to further aspects disclosed herein, the method can optionally include causing the ultrasound transducer to emit acoustic energy at an ablating level such that the ultrasound transducer creates a second circumferential lesion about the blood vessel without repositioning the ablation catheter, wherein the second circumferential lesion is spaced apart from the first circumferential lesion along a length of the blood vessel. For example, the acoustic energy emitted at the ablating level can be electronically steered to a location of the second circumferential lesion. Alternatively, the ultrasound transducer can slide along a length of the distal region prior to causing the ultrasound transducer to emit acoustic energy at an ablating level such that the ultrasound transducer creates a second circumferential lesion about the blood vessel. It is also contemplated that the second circumferential lesion can be at a common position along a length of the blood vessel, but at a different depth into the tissue, relative to the first circumferential lesion. In still other embodiments, the second circumferential lesion can overlap the first circumferential lesion.

The foregoing and other aspects, features, details, utilities, and advantages of the present invention will be apparent from reading the following description and claims, and from reviewing the accompanying drawings.

### BRIEF DESCRIPTION OF THE DRAWINGS

Figure 1 schematically depicts an ablation and lesion feedback system according to aspects of the instant disclosure.
Figure 2 is a schematic view of the distal region of an embodiment of the ablation and lesion feedback catheter shown in Figure 1, partially cut away to show details of the interior thereof.
Figure 3A is a cross-sectional view taken along line 3-3 in Figure 2 of a first exemplary embodiment of an ablation and lesion feedback catheter.
Figure 3B is a cross-sectional view taken along line 3-3 in Figure 2 of a second exemplary embodiment of an ablation and lesion feedback catheter.
Figure 4 is a partially cut away view of the distal region of another embodiment of the ablation and lesion feedback catheter shown in Figure 1.
Figure 5 is a view from inside a blood vessel, looking proximally along the body of the ablation and lesion feedback catheter shown in Figure 1, with the balloon expanded.
Figure 6 is a side view of an ablation and lesion feedback catheter according to another exemplary embodiment, showing the catheter deployed in a blood vessel to create a circumferential lesion.
Figure 7 is a side view of an ablation and lesion feedback catheter according to still another exemplary embodiment, showing the catheter deployed in a blood vessel to create a circumferential lesion.

### DETAILED DESCRIPTION

The present disclosure provides medical devices, such as catheters, suitable for use in creating an ablation lesion about the circumference of a blood vessel. Such a lesion is referred to herein as a "circumferential lesion," and includes both closed-ended lesions (*e.g.,* a circular lesion that loops around the vessel wall a single time) and open-ended lesions (*e.g*., a helical lesion that loops around the vessel wall multiple times). For purposes of illustration, several exemplary embodiments will be described herein in detail in connection with a system that utilizes ultrasound energy for both ablation (*e.g*., HIFU ablation) and imaging/monitoring (*e.g*., ultrasound imaging/monitoring) in the context of a PV isolation procedure. It should be understood, however, that the teachings herein can be utilized in other contexts, such as renal denervation, and/or with other electromagnetic ablation modalities, such as radiofrequency (RF) ablation, laser ablation, and the like. Similarly, it should be understood that the teachings herein can be utilized with other imaging/monitoring modalities.

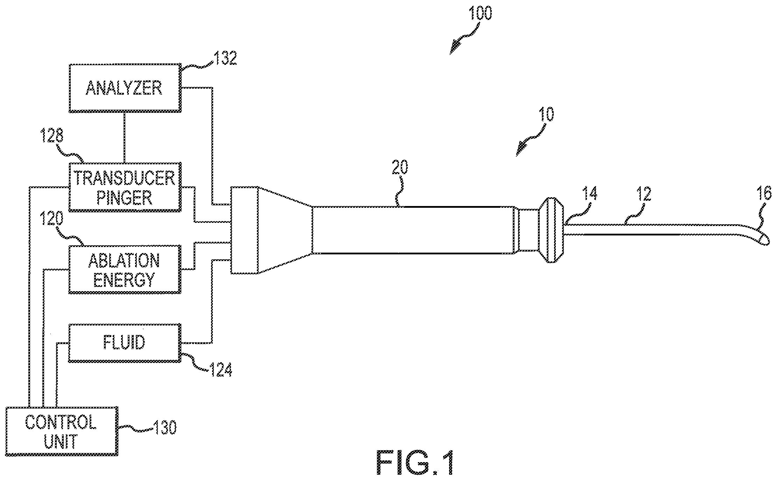

Figure 1 is a schematic diagram of an ablation and lesion feedback system 100 including an exemplary catheter 10. As shown in Figure 1, catheter 10 generally includes an elongate, hollow, and flexible tubular body 12 having a proximal end 14 and a distal region 16.

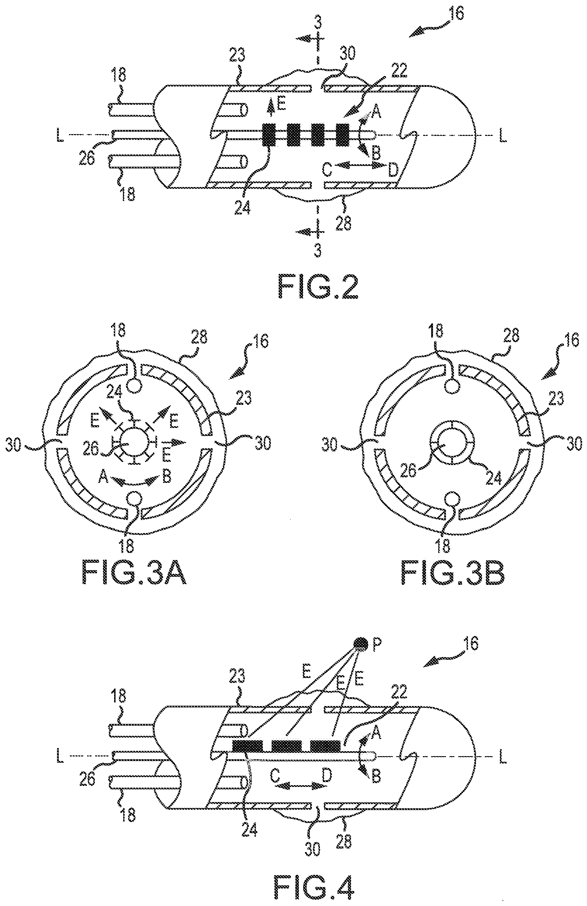

Tubular body 12 includes one or more lumens 18 (not visible in Figure 1, but visible, *inter alia,* in Figure 2). Although particular numbers and configurations of lumens 1 8 are depicted in certain figures for purposes of illustration, it should be understood that any number and configuration of lumens 18 can be used without departing from the scope of the instant teachings. As the person of ordinary skill in the art will appreciate, tubular body 12 can also contain electrical interconnect wires, pull-wires, and the like.

Proximal end 14 of tubular body 12 is attached to a catheter control handle 20. Catheter control handle 20 can include, for example, an actuator (not shown) coupled to suitable structure (*e.g*., pull wires and/or pull rings) within tubular body 12 in order to effect the deflection of distal end 16 in one or more bending planes. It can also include electrical power, electrical signal, and/or fluidic connections to additional components of ablation system 100 as discussed in further detail below.

Figure 2 schematically depicts the interior of distal region 16 of catheter body 12, including an ablation element 22 disposed therein. Ablation element 22 can be coupled to an ablation energy generator 120, shown schematically in Figure 1, and is oriented such that it emits ablative energy outwardly from the longitudinal axis L of catheter body 12 towards the wall 23 of catheter body 12 (*e.g.,* along arrows "E," shown in Figures 2 and 3).

In embodiments, ablation element 22 is an acoustic ablation element, which in turn can include one or more ultrasound transducers 24, each of which, as will be familiar to those of ordinary skill in the art, can incorporate one or more ultrasound elements. Advantageously, ultrasound transducers 24 can be used both for ablation and for imaging and/or monitoring nearby tissue. For example, in an ablation modality, control unit 130 can couple ultrasound transducers 24 to ablation energy generator 120 and drive them to emit HIFU energy to ablate surrounding tissue. In an imaging or monitoring modality, on the other hand, control unit 130 can couple ultrasound transducers 24 to transducer pinger 128 and drive them to emit ultrasound energy at a suitable level to image and/or monitor surrounding tissue, based on acoustic energy echoes received.

In some embodiments, therefore, control unit 130 can be configured to carry out duty cycling or synchronization for both ablation and pinging. (The term "pinging" is used herein to mean transmitting acoustic energy and then receiving the reflected or echoed acoustic energy, such as in the case of imaging and/or monitoring the nearby tissue.) An acoustic pinger echo analyzer or acoustic receiver 132 can be provided to condition and analyze the data collected when ultrasound transducers 24 are in an imaging or monitoring modality, for example to provide lesion feedback. The information can be presented to a practitioner (*e,g.,* using a graphical user interface) to provide real-time assessment of the ablation target, the ablation process, and/or the ablation result. The information may additionally or alternatively be used by the system itself without operator intervention, for example as input to a feedback control loop controlling ablation power and/or irrigant cooling to avoid steam pops and/or to achieve a desired lesion depth.

Various physical configurations of ultrasound transducers 24 are contemplated for use as ablation element 22. Two such contemplated configurations are "linear arrays" and "circumferential arrays," each of which will be discussed in further detail below, though it should be understood that these two configurations are not mutually exclusive (that is, the disclosure contemplates a configuration of ultrasound transducers that is a combination linear and circumferential array).

In a linear array, a plurality of ultrasound transducers 24 are generally arranged extending along the longitudinal axis L of catheter body 12, such as shown in Figure 2. It should be understood that, notwithstanding the depiction in Figure 2, any number of ultrasound transducers 24 (each including any number of ultrasound elements) could be employed without departing from the scope of the teachings herein regarding linear arrays.

In a circumferential array, a plurality of ultrasound transducers 24 are generally arranged about a circumference of catheter body 12, such as shown in Figure 3A. Once again, it should be understood that, notwithstanding the depiction in Figure 3A, any number of ultrasound transducers 24 (each including any number of ultrasound elements) could be employed without departing from the scope of the instant teachings regarding circumferential arrays.

Other arrangements of ultrasound transducers 24 can be employed without departing from the instant teachings. For example, in the embodiment depicted in Figure 3B, each ultrasound transducer 24 is a cylindrical transducer (which, as discussed above, can include one or more ultrasound elements). It should also be understood that a single embodiment can include ultrasound transducers arranged in both linear array and circumferential array (*e.g*., as shown in Figures 2 and 3A).

Functionally, it is contemplated that ultrasound transducers 24 can be omnidirectional (*i.e*., emitting ultrasound simultaneously at 360 degrees around the longitudinal axis), unidirectional, or a combination thereof. For example, it would be desirable to use an omnidirectional ultrasound transducer 24 in the embodiment depicted in Figure 3B, which allows for HIFU energy for ablation to be emitted at 360 degrees simultaneously, without the need to reposition or rotate catheter 10 within the vessel being ablated.

In some embodiments, ultrasound transducers 24 are mounted such that they can rotate about the longitudinal axis L of catheter body 12 (*e.g*., following arrow A-B in Figures 3A and 4), for example by attaching ultrasound transducers 24 to a rotatable shaft 26. This arrangement is particularly desirable for unidirectional ultrasound transducers 24, as it facilitates the creation of a circumferential lesion without requiring the catheter 10 itself to be repositioned and/or rotated within the vessel.

For example, Figure 4 depicts a plurality of unidirectional ultrasound transducers 24 arranged on shaft 26 in a linear array. The energy E emitted by ultrasound transducers 24 is focused (mechanically and/or electronically) at a point P on the wall of the blood vessel or PV (for the sake of clarity, the PV is not shown in Figure 4). Thus, a complete or nearly complete revolution of shaft 26 about arrow A-B is required to make a complete circumferential lesion (*e.g*., a series of points P, extending 360 degrees about the circumference of the PV, sufficiently close to and/or overlapping each other in a manner that is effectively continuous for purposes of blocking the conduction of electricity across the lesion). In other words, by rotating shaft 26, ultrasound transducers 24 can be repositioned to facilitate ablation, imaging, and/or monitoring about the entire circumference of a blood vessel within which distal region 16 is positioned without the need to reposition catheter 10 itself.

It should be understood that, as used herein, the term "rotate" is not limited to continuous, 360 degree revolutions of shaft 26 about the longitudinal axis of body 12. Rather, the term "rotate" is used broadly to encompass not only continuous revolutions of shaft 26, but also various steps and/or oscillatory arcs of shaft 26.

Shaft 26 can be driven by a stepper motor or servo motor, such that the various rotational orientations of ultrasound transducers 24 can be known (*e.g*., by a motor-integrated encoder). Alternatively, a rotary encoder, which can be mechanical, optical, magnetic, capacitive, or of any other suitable technology can be used to output the rotational attitude of ultrasound transducers 24. In still other embodiments, a sensor (*e.g.,* a magnetic coil) can be provided on shaft 26 in order to determine the rotational attitude of ultrasound transducers 24. This information can, in turn, be used, such as by control unit 130 and/or analyzer 132, in the ablation, imaging, and/or monitoring procedure. For example, United States provisional application no. 62/100,756 describes the use of a plurality of two-dimensional image slices, each associated with a particular rotational attitude, to assemble a three-dimensional volumetric image of a blood vessel with the corresponding circumferential lesion(s).

According to other aspects of the disclosure, shaft 26 is impeller-driven, such as described in United States application no. 12/347,116.

In still further embodiments, a complete circumferential lesion can be created, without rotating shaft 26, from a plurality of unidirectional ultrasound transducers 24 arranged in a circumferential array. For example, with reference to Figure 3A, a complete circumferential lesion can be created by sequentially or simultaneously activating various ultrasound transducers 24 and/or subgroups thereof (*e.g*., first activating 12 o'clock through 3 o'clock; then activating 3 o'clock through 6 o'clock; then activating 6 o'clock through 9 o'clock; finally activating 9 o'clock through 12 o'clock). The ultrasound transducers 24 and/or subgroups thereof can be adjacent and/or overlapping. In general, however, by "walking" unidirectional ultrasound energy around the circumference of catheter body 12 in this manner, the need to rotate shaft 26 can be minimized, and perhaps eliminated, while still affording fall circumferential coverage for ablation, imaging, and/or monitoring, again without the need to reposition catheter 10 itself.

According to additional aspects of the instant disclosure, ultrasound transducers 24 are mounted such that they can move back and forth along the longitudinal axis of catheter body 12 (*e.g*., parallel to arrow C-D in Figures 2 and 4; into and out of the plane of the page in Figures 3A and 3B), for example via the use of a finger slider or other suitable actuator on the handle of catheter 10 that pushes or pulls shaft 26 within catheter body 12. This allows for the ablation, imaging, and/or monitoring of different points along the length of the blood vessel (*e.g*., more proximal vs. more distal) within which distal region 16 is positioned, again without the need to reposition catheter 10 itself. This objective (that is, the ablation, imaging and/or monitoring of different points along the length of the surrounding blood vessel) can also be achieved via beam steering (that is, electronically steering the focus of the ultrasonic energy emitted by ultrasound transducers 24 such that point P in Figure 4 moves distally or proximally along longitudinal axis L), for example under the direction of practitioner input and/or pre-programmed protocols within control unit 130.

A balloon 28 is positioned about distal region 16. The interior of balloon 28 is in communication with lumens 18, for example through vias 30. Thus, a fluid (*e.g*., saline) can be provided from fluid source 124 (shown in Figure 1), through lumen(s) 18, in order to expand balloon 28 outwardly from an outer surface of catheter body 12. Advantageously, the fluid can also help cool ultrasound transducers 24, facilitate energy transmission from ultrasound transducers 24 to adjacent tissue, and/or serve acoustic matching purposes.

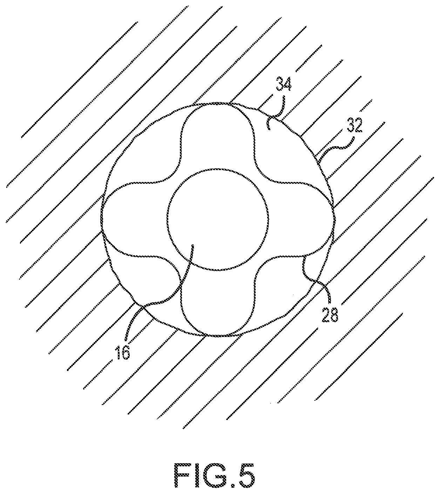

As shown in Figure 5, when expanded, balloon 28 is shaped such that it can engage the interior wall of a blood vessel 32 in a manner that holds catheter 10 stable relative to vessel 32 without occluding vessel 32 (Figure 5 shows catheter 10 end on, looking proximally from a point within vessel 32). As used herein, vessel 32 is "occluded" if it is sufficiently obstructed to impede the flow of blood in a manner detrimental to the patient. Thus, while the merest of obstructions likely will not constitute an "occlusion," it is also not required that the vessel be completely obstructed before being deemed "occluded" within the meaning of the instant disclosure.

It should be understood, therefore, that, when expanded, balloon 28 will press against the interior wall of blood vessel 32 along sufficient interfacing surface to hold catheter 10 stable, but while retaining interstices 34 between the exterior surface of balloon 28 and the interior wall of blood vessel 32 to permit perfusion. One suitable expanded shape for balloon 28 is a cross-shape, as shown in Figure 5. Other expanded balloon shapes are also contemplated.

It is also desirable for balloon 28 to be positioned such that ablation element 22 (*e.g*., ultrasound transducers 24) are positioned therein.

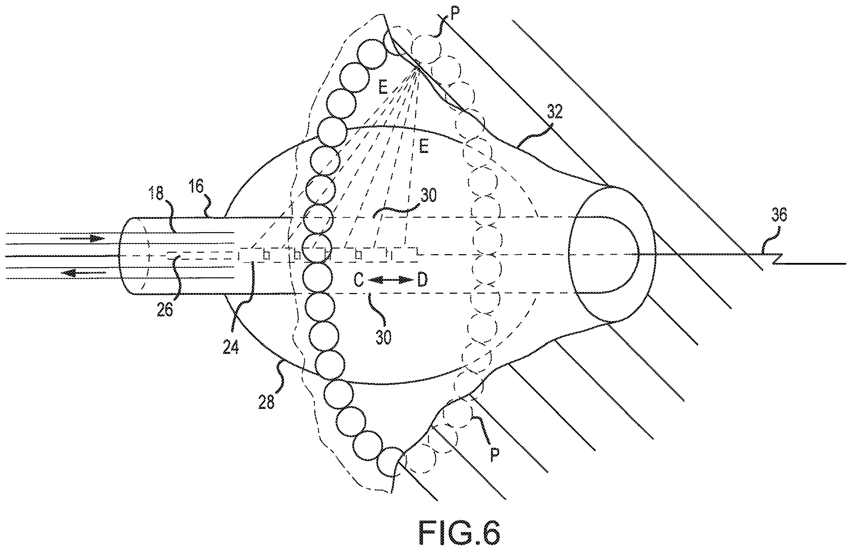

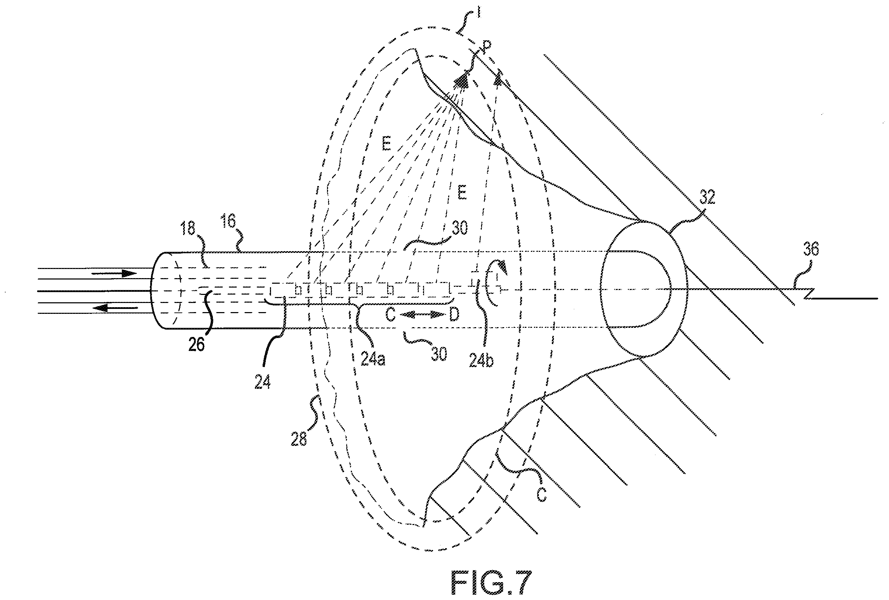

Uses of exemplary embodiments of catheter 10 are depicted in Figures 6 and 7. In Figures 6 and 7, catheter 10 has been introduced into a patient's vasculature and advanced, for example over guidewire 36, until it is positioned at the desired location for diagnosis and/or treatment, such as with distal region 16 positioned within a pulmonary vein 32. Once positioned, balloon 28 can be expanded so that catheter 10 is stably anchored in pulmonary vein 32. As described above, the transverse cross-section of Figures 6 and 7 (that is, with balloon 28 expanded) resembles Figure 5.

Ultrasound transducers 24 can then be activated to image pulmonary vein 32. Based on the image, the practitioner can select various ablation parameters, such as lesion location, lesion depth, ablation power, ablation time, the rotational speed of shaft 26, and the like.

Once pulmonary vein 32 is imaged, ultrasound transducers 24 can be activated, e.g., according to user-defined parameters as discussed above, to emit acoustic energy at a level sufficient to ablate the adjacent tissue, for example under excitation by ablation energy generator 120.

As shown in Figure 6, and in similar fashion to Figure 4, the energy E emitted from a plurality of ultrasound transducers 24 (shown in dashed lines) arranged in a linear array is focused, electronically and/or mechanically, to a target point P on the wall of pulmonary vein 32. This creates a spot lesion at point P. As shown schematically in Figure 6, a series of spot lesions at a plurality of points P around pulmonary vein 32, which can be created by rotating shaft 26 and/or through the use of omnidirectional transducers 24, results in the creation of a circumferential lesion (that is, a lesion that blocks electrical conduction along the length of pulmonary vein 32).

In embodiments where ultrasound transducers 24 are unidirectional (that is, they emit and receive acoustic energy along a single plane), the series of spot lesions at the plurality of points P can be created by rotating ultrasound transducers 24 via shaft 26 and/or by the use of a circumferential array of ultrasound transducers (*e.g*., as shown in Figure 3A).

In other embodiments, where ultrasound transducers 24 are omnidirectional (that is, they emit and receive acoustic energy at 360 degrees, such as shown in Figure 3B), all or substantially all of the spot lesions at the plurality of points B can be created at one time, minimizing, and potentially eliminating, the need to rotate ultrasound transducers 24/shaft 26.

Similar to Figure 6, Figure 7 depicts an embodiment of catheter 10 that includes a plurality of ultrasound transducers 24 mounted to shaft 26. A subset of these ultrasound transducers, designated 24a, are operable primarily in an ablation modality, while an additional ultrasound transducer, designated 24b, is operable primarily in an imaging modality. Ultrasound transducers 24a and 24b can incorporate any of the teachings disclosed herein in any permutation. For example, ultrasound transducers 24a can be electronically and/or mechanically focused omnidirectional transducers that, when activated, are able to create all or substantially all of a circumferential lesion C without requiring rotation. As another example, ultrasound transducer 24b may be a rotatable unidirectional ultrasound transducer that, when rotated about shaft 26, images in plane I, and circumferential lesion C can be in-plane with imaging plane I.

In general, however, catheter 10 itself (*e.g*., as depicted in Figures 6 and 7) need not be moved and/or rotated relative to pulmonary vein 32 to complete the circumferential lesion.

The position of the first circumferential lesion along the length of the blood vessel can be dictated by the physical positioning of ultrasound transducers 24 within distal region 16 (*e.g*., the point or points along the length of catheter body 12 at which ultrasound transducers 24 are positioned) and/or by electronic beam steering. Thus, a second (or additional) circumferential lesion(s) can be formed in the vessel wall, without repositioning catheter 10 relative to pulmonary vein 32, for example by changing the position of ultrasound transducers 24 along the length of catheter body 12 (*e.g.,* by pushing and/or pulling shaft 26 along arrow C-D) and/or by changing the operating parameters of ultrasound transducers 24 to electronically steer the acoustic energy emitted thereby so that points P, and thus circumferential lesion C, move distally or proximally, as desired.

It is also contemplated that an additional circumferential lesion(s) can be created at a common position along the length of catheter body 12, but at a different depth in the tissue relative to the first circumferential lesion.

In still other embodiments, the additional lesion(s) can overlap, in whole or in part, with the first circumferential lesion.

Similar procedures, at lower power for the emitted acoustic energy (*e.g*., under excitation from transducer pinger 128), can be followed to utilize ultrasound transducers 24 to monitor the formation of a lesion in the vessel wall and/or to image the vessel (as noted above, imaging will often precede ablation). In addition, ablation, imaging, and/or monitoring can be interleaved (*e.g*., 10 seconds of ablation, followed by 2-3 seconds of imaging and/or monitoring).

The foregoing methods can be carried out by one or more processors incorporated into control unit 130 and/or analyzer 132. As used herein, the term "processor" refers to not only a single central processing unit ("CPU"), but also to a plurality of processing units, commonly referred to as a parallel processing environment. It should also be understood that the methods disclosed herein can be hardware and/or software implemented.

Although several embodiments of this invention have been described above with a certain degree of particularity, those skilled in the art could make numerous alterations to the disclosed embodiments without departing from the scope of this invention.

All directional references (e.g., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting. Changes in detail or structure may be made without departing from the scope of the invention as defined in the appended claims.

## Claims

1. An ablation system (100), comprising
an elongate catheter body (12) having a lumen (18), the elongate catheter body (12) including a distal region (16),
an ablation element (22) disposed within the distal region (16), wherein the ablation element (22) is configured to emit ablative energy outwardly relative to a longitudinal axis of the elongate catheter body (12), and
a balloon (28) positioned about the distal region (16) and defining an interior in communication with the lumen (18),
wherein the balloon (28) is expandable outwardly from an outer surface of the elongate catheter body (12), and wherein, when the balloon (28) is expanded outwardly from the outer surface of the elongate catheter body (12), an exterior surface of the balloon (28) is shaped to stably engage an interior wall of a blood vessel while retaining interstices (34) between the exterior surface of the balloon (28) and the interior wall of the blood vessel, thereby to avoid occluding blood flow through the blood vessel,
wherein the ablation element is further configured to operate in an imaging mode,
wherein the ablation element (22) is an ultrasound transducer (24) disposed within the distal region (16), wherein the ultrasound transducer (24) is configured to emit acoustic energy outwardly relative to a longitudinal axis of the elongate catheter body (12) and to receive acoustic energy responsive to the emitted acoustic energy, and
wherein the system further comprises a control unit (130) configured to operate the ultrasound transducer (24) in an ablating mode, wherein the ultrasound transducer (24) emits acoustic energy at an ablation level, and in the imaging mode, wherein the ultrasound transducer (24) emits acoustic energy at an imaging level and receives acoustic energy echoes from adjacent tissue.

2. The system according to claim 1, wherein the control unit (130) is further configured to electronically steer a focus of the acoustic energy emitted by the ultrasound transducer (24).

3. The system according to claim 1 or 2, wherein the ultrasound transducer (24) comprises a circumferential array of acoustic elements.

4. The system according to any one of claims 1 to 3, wherein the ultrasound transducer (24) is mounted within the distal region (16) such that the ultrasound transducer (24) can rotate about a longitudinal axis of the elongate catheter body (12).

5. The system according to any one of claims 1 to 4, wherein the ultrasound transducer (24) comprises an omnidirectional ultrasound transducer.

6. The system according to any one of claims 1 to 5, wherein the control unit (130) is further configured to operate the ultrasound transducer (24) in the ablating mode to create a circumferential lesion in a wall of a blood vessel surrounding the distal region.

7. The system according to any one of claims 1 to 6, wherein
the ultrasound transducer (24) comprises
a linear array of omnidirectional acoustic elements, and
a unidirectional acoustic element mounted within the distal region (16) such that the unidirectional acoustic element can rotate about a longitudinal axis of the elongate catheter body (12), and
the control unit (130) is configured to operate the linear array of omnidirectional acoustic elements in the ablating mode and to operate the unidirectional acoustic element in the imaging mode.

8. The system according to any one of claims 1 to 7,
wherein the ultrasound transducer (24) comprises a plurality of ultrasound transducers (24) arranged in a circumferential array, and/or
wherein the ultrasound transducer (24) comprises a plurality of ultrasound transducers (24) arranged in a linear array.

9. The system according to any one of claims 1 to 8, wherein the ultrasound transducer is disposed within the balloon (28).

10. The system according to any one of claims 1 to 9, wherein the balloon (28) has a cross-shaped transverse cross-section when expanded.

## Patentansprüche

1. Ablationssystem (100), mit
einem länglichen Katheterkörper (12), der ein Lumen (18) aufweist, bei dem der längliche Katheterkörper (12) einen distalen Bereich (16) aufweist,
einem Ablationselement (22), das innerhalb des distalen Bereiches (16) angeordnet ist, bei dem das Ablationselement (22) dazu konfiguriert ist, Ablationsenergie nach außen relativ zu einer Längsachse des länglichen Katheterkörpers (12) zu emittieren, und
einem Ballon (28), der um den distalen Bereich (16) positioniert ist und ein Innenraum in Verbindung mit dem Lumen (18) definiert,
bei dem der Ballon (28) nach außen von einer äußeren Oberfläche des länglichen Katheterkörpers (12) expandierbar ist, und bei dem, wenn der Ballon (28) nach außen von der äußeren Oberfläche des länglichen Katheterkörpers (12) expandiert ist, eine Außenoberfläche des Ballons (28) derart ausgeformt ist, dass sie mit einer Innenwand eines Blutgefäßes stabil in Eingriff steht, während Zwischenräume (34) zwischen der äußeren Oberfläche des Ballons (28) und der Innenwand des Blutgefäßes beibehalten werden, und dadurch ein Verstopfen des Blutflusses durch das Blutgefäß vermieden wird,
bei dem das Ablationselement ferner dazu konfiguriert ist, in einem Abbildungsmodus zu arbeiten,
bei dem das Ablationselement (22) ein Ultraschallwandler (24) ist, der innerhalb des distalen Bereiches (16) angeordnet ist, bei dem der Ultraschallwandler (24) dazu konfiguriert ist, akustische Energie nach außen relativ zu einer Längsachse des länglichen Katheterkörpers (12) zu emittieren und akustische Energie in Antwort auf die emittierte akustische Energie zu empfangen, und
bei dem das System ferner eine Steuerungseinheit (130) aufweist, die zum Betreiben des Ultraschallwandlers (24) in einem Ablationsmodus, bei dem der Ultraschallwandler (24) akustische Energie bei einem Abbildungslevel emittiert, und in dem Abbildungsmodus konfiguriert ist, bei welchem der Ultraschallwandler (24) akustische Energie bei einem Abbildungslevel emittiert und akustische Energieechos von benachbartem Gewebe empfängt.

2. System nach Anspruch 1, bei dem die Steuerungseinheit (130) ferner dazu konfiguriert ist, einen Schwerpunkt der akustischen Energie, die durch den Ultraschallwandler (24) emittiert wird, elektronisch zu steuern.

3. System nach Anspruch 1 oder 2, bei dem der Ultraschallwandler (24) eine umfängliche Anordnung von akustischen Elementen aufweist.

4. System nach einem der Ansprüche 1 bis 3, bei dem der Ultraschallwandler (24) innerhalb des distalen Bereiches (16) derart montiert ist, dass der Ultraschallwandler (24) um eine Längsachse des länglichen Katheterkörpers (12) drehen kann.

5. System nach einem der Ansprüche 1 bis 4, bei dem der Ultraschallwandler (24) einen omnidirektionalen Ultraschallwandler aufweist.

6. System nach einem der Ansprüche 1 bis 5, bei dem die Steuerungseinheit (130) ferner dazu konfiguriert ist, den Ultraschallwandler (24) in dem Ablationsmodus zum Erzeugen einer umfänglichen Läsion in einer Wand eines Blutgefäßes, das den distalen Bereich umgibt, zu betreiben.

7. System nach einem der Ansprüche 1 bis 6, bei dem
der Ultraschallwandler (24)
eine lineare Anordnung von omnidirektionalen akustischen Elementen, und
ein unidirektionales akustisches Element aufweist, das innerhalb des distalen Bereiches (16) montiert ist, so dass das unidirektionale akustische Element um eine Längsachse des länglichen Katheterkörpers (12) drehen kann, und
die Steuerungseinheit (130) dazu konfiguriert, die lineare Anordnung des omnidirektionalen akustischen Elements in dem Ablationsmodus zu betreiben und das unidirektionale akustische Element in dem Abbildungsmodus zu betreiben.

8. System nach einem der Ansprüche 1 bis 7,
bei dem der Ultraschallwandler (24) eine Mehrzahl von Ultraschallwandlern (24) aufweist, die in einer umfänglichen Anordnung angeordnet sind, und/oder
bei dem der Ultraschallwandler (24) eine Mehrzahl von Ultraschallwandlern (24) aufweist, die in einer linearen Anordnung angeordnet sind.

9. System nach einem der Ansprüche 1 bis 8, bei dem der Ultraschallwandler innerhalb des Ballons (28) angeordnet ist.

10. System nach einem der Ansprüche 1 bis 9, bei dem der expandierte Ballon (28) einen kreuzförmigen Querschnittsabschnitt aufweist.

## Revendications

1. Système d'ablation (100), comprenant
un corps de cathéter allongé (12) ayant une lumière (18), le corps de cathéter allongé (12) comprenant une région distale (16),
un élément d'ablation (22) disposé dans la région distale (16), dans lequel l'élément d'ablation (22) est configuré pour émettre une énergie ablative vers l'extérieur par rapport à un axe longitudinal du corps de cathéter allongé (12), et
un ballon (28) positionné autour de la région distale (16) et définissant un intérieur en communication avec la lumière (18),
dans lequel le ballon (28) est extensible vers l'extérieur à partir d'une surface extérieure du corps de cathéter allongé (12), et dans lequel, lorsque le ballon (28) est extensible vers l'extérieur à partir de la surface extérieure du corps de cathéter allongé (12), une surface extérieure du ballon (28) est formée pour s'engager de manière stable dans une paroi intérieure d'un vaisseau sanguin tout en maintenant des interstices (34) entre la surface extérieure du ballon (28) et la paroi intérieure du vaisseau sanguin, pour empêcher ainsi une occlusion d'écoulement de sang à travers le vaisseau sanguin,
dans lequel l'élément d'ablation est en outre configuré pour fonctionner dans un mode d'imagerie,
dans lequel l'élément d'ablation (22) est un transducteur à ultrasons (24) disposé dans la région distale (16), dans lequel le transducteur à ultrasons (24) est configuré pour émettre de l'énergie acoustique vers l'extérieur par rapport à un axe longitudinal du corps de cathéter allongé (12) et pour recevoir une énergie acoustique répondant à l'énergie acoustique émise, et
dans lequel le système comprend en outre une unité de commande (130) configurée pour faire fonctionner le transducteur à ultrasons (24) dans un mode d'ablation, dans lequel le transducteur à ultrasons (24) émet une énergie acoustique à un niveau d'ablation, et dans le mode d'imagerie, dans lequel le transducteur à ultrasons (24) émet une énergie acoustique au niveau d'imagerie et reçoit une énergie acoustique provenant de tissu adjacent.

2. Système selon la revendication 1, dans lequel l'unité de commande (130) est en outre configurée pour diriger électroniquement un foyer de l'énergie acoustique émise par le transducteur à ultrasons (24).

3. Système selon la revendication 1 ou 2, dans lequel le transducteur à ultrasons (24) comprend un réseau circonférentiel d'éléments acoustiques.

4. Système selon l'une quelconque des revendications 1 à 3, dans lequel le transducteur à ultrasons (24) est monté dans la région distale (16) de telle sorte que le transducteur à ultrasons (24) peut tourner autour d'un axe longitudinal du corps de cathéter allongé (12).

5. Système selon l'une quelconque des revendications 1 à 4, dans lequel le transducteur à ultrasons (24) comprend un transducteur à ultrasons omnidirectionnel.

6. Système selon l'une quelconque des revendications 1 à 5, dans lequel l'unité de commande (130) est en outre configurée pour faire fonctionner le transducteur à ultrasons (24) en mode ablation pour créer une lésion circonférentielle dans une paroi d'un vaisseau sanguin entourant la région distale.

7. Système selon l'une quelconque des revendications 1 à 6, dans lequel
le transducteur à ultrasons (24) comprend
un réseau linéaire d'éléments acoustiques omnidirectionnels, et
un élément acoustique unidirectionnel monté dans la région distale (16) de telle sorte que l'élément acoustique unidirectionnel peut tourner autour d'un axe longitudinal du corps de cathéter allongé (12), et
l'unité de commande (130) est configurée pour faire fonctionner le réseau linéaire d'éléments acoustiques omnidirectionnels dans le mode ablation et pour faire fonctionner l'élément acoustique unidirectionnel dans le mode imagerie.

8. Système selon l'une quelconque des revendications 1 à 7,
dans lequel le transducteur à ultrasons (24) comprend une pluralité de transducteurs à ultrasons (24) disposés dans un réseau circonférentiel, et/ou
dans lequel le transducteur à ultrasons (24) comprend une pluralité de transducteurs à ultrasons (24) disposés en réseau linéaire.

9. Système selon l'une quelconque des revendications 1 à 8, dans lequel le transducteur à ultrasons est disposé dans le ballon (28).

10. Système selon l'une quelconque des revendications 1 à 9, dans lequel le ballon (28) présente une section transversale en forme de croix lorsqu'il est étendu.
